# EUROPEAN PATENT SPECIFICATION

(11) **EP 4 297 677 B1**
(45) Date of publication and mention of the grant of the patent: **21.10.2026**
(21) Application number: 22710838.8
(22) Date of filing: 25.02.2022
(51) Int. Cl.: A61B 18/12

(54) **PULSED ELECTRIC FIELD (PEF) INDEX**
INDEX FÜR GEPULSTES ELEKTRISCHES FELD (PEF)
INDICE DE CHAMP ÉLECTRIQUE PULSÉ (PEF)

(30) Priority: 26.02.2021 US 202163154259 P; 23.02.2022 US 202217678102
(43) Date of publication of application: 03.01.2024
(73) Proprietor: Medtronic, Inc., Minneapolis, Minnesota 55432 (US)
(72) Inventor: HOWARD, Brian T., Minneapolis, Minnesota 55432 (US); SCHMIDT, Megan M., Minneapolis, Minnesota 55432 (US); MATTISON, Lars M., Minneapolis, Minnesota 55432 (US)
(74) Representative: Zimmermann & Partner Patentanwälte mbB
(86) International application number: PCT/US2022/017818
(87) International publication number: WO 2022/182939

(56) References cited:
- EP-A2- 2 665 433
- US-A1- 2019 254 735

## Description

### FIELD

The present technology is generally related to pulsed electric field (PEF) energy delivery systems and methods, and in particular, an index to determine a completeness of a particular PEF treatment.

### BACKGROUND

Medical procedures such as cardiac ablation using one or more energy modalities are frequently used to treat conditions such as atrial fibrillation and ventricular tachycardia. Standard treatment may use radiofrequency (RF) ablation which involves heating target tissue to cause cell death and thus change conduction pathways in the heart to treat a disease state in a patient. Excessive application of RF energy can result in collateral damage. Treatment may be quantified by measurements such as temperature rise, contact force, total energy, impedance, or electrogram (EGM) waveforms to indicate an amount of thermal energy conveyed to and/or retained by the target tissue. In contrast, pulsed electric field (PEF) ablation involves application of an electric field to disrupt cellular membranes. The electric fields are delivered in short bursts. Disruption of the cell membrane results in the desired cell death. Cell death may result in impedance changes because of the liberated exchange of ions through the permeabilized cell membranes. Different cells types may also be affected differently by PEF energy such that collateral structures may be unaffected by the PEF energy as they would be by the temperature rise induced by RF ablation. Note that temperature rise may also occur for PEF ablation but to a lesser extent. The electric field in PEF ablation may be established between conductive elements such as electrodes and conducts current through the target tissue acting as a resistive medium which necessarily results in energy dissipation or temperature rise in the tissue. This thermal energy is generally less than the thermal energy generated by RF ablation but still presents a risk to collateral structures, especially with successive applications.

In addition to being a different way of inducing cell death, a PEF is also generally very fast to apply. This makes normal measures of therapeutic endpoints generally inapplicable or incomplete to describe what the applied therapy has achieved. Ablation with PEF may be effective without imparting sufficient energy to cause thermal damage, which is an identified risk of radiofrequency ablation. Generally, to effect a larger region of targeted tissue, application of higher energies for PEF may be used. However, there is a tradeoff between effecting a larger tissue area versus a corresponding temperature rise in the tissue. Mitigations to this effect such as identifying an appropriate endpoint to therapy application may increase the energy that may be delivered with PEF while reducing the risk of thermal damage. Document US 2019/254735 A1 relates to focal pulsed field ablation devices and methods, wherein a control unit may compare recorded EGMs to a threshold EGM value and selectively activate or deactivate individual energy return electrodes based on the comparision. Document EP 2 665 433 A2 relates to devices for treatment of tissue.

### SUMMARY

The invention is defined in claim 1. Any methods disclosed hereinafter do not form part of the scope of the invention, and are mentioned for illustrative purposes only. The techniques of this disclosure generally relate to pulsed electric field ablation.

In one aspect, a medical system includes a generator configured to generate pulsed electric field (PEF) energy. The generator is configured to generate at least one pulsed electric field (PEF) pulse to be delivered to at least one electrode of a plurality of electrodes, the at least one electrode being at a distal end of a PEF ablation catheter and being positionable in proximity to a target region of tissue to be ablated. The generator is further configured to determine an index of completeness indicative of a completeness of ablation of the target region of tissue based at least in part on a change in a parameter compared to an expected change in the parameter, the change in the parameter being caused at least in part on an extent of ablation of the target region.

In some embodiments, the processing circuitry is configured to set a predetermined depth, size, or width of lesion formation, and the index of completeness is a ratio of the achieved depth, size, or width during delivery of PEF energy to the target tissue to the predetermined depth, size, or width of lesion formation.

In some embodiments, the processing circuitry is configured to be preprogrammed with a threshold for an acceptable ratio of completeness, and the processing circuitry is configured to terminate the generation of PEF energy to the medical device if the threshold for an acceptable ratio of completeness is achieved.

In some embodiments, the processing circuitry is configured to generate an alert and/or engage additional functionalities such as discontinuation of therapy if the determined ratio of completeness exceeds a predetermined acceptable ratio of completeness threshold.

In some embodiments, the system further includes a display in communication with the processing circuitry and the medical device, and the processing circuitry is configured to cause display of the index of completeness for at least one from the group consisting of: each of the plurality of electrodes, subsets of electrodes, and a totality of the electrodes.

In some embodiments, the processing circuitry is configured to cause display of an area in proximity to at least one of the plurality of electrodes treated with PEF energy.

In some embodiments, the processing circuitry is configured to display on a display an indicator that is at least one of color-coded of variable opacity to indicate the index of completeness.

In some embodiments, the processing circuitry is configured to measure impedance of at least one from the group consisting of each electrode, between adjacent electrodes, between one of the plurality of electrodes and a ground electrode.

In some embodiments, the processing circuitry is configured to measure the temperature of the plurality of electrodes.

In some embodiments, the index of completeness is a measure based on at least one of depth, volume, width, transmurality, and continuity of a lesion created.

In some embodiments, determining the index of completeness includes determining $C = k \times \frac{\left({T}_{f}-{T}_{i}\right)}{Δ {T}_{n}}$, where "k" is a constant, "*T_{f}"* is the final temperature, "*Tᵢ*" is the initial temperature, and *"*Δ*Tₙ"* is the expected change in temperature.

In some embodiments, determining the index of completeness includes determining $C = k \times \frac{\left({Z}_{f}-{Z}_{i}\right)}{Δ {Z}_{n}}$, where "k" is a constant and "Z_{f}" is the final impedance, "Zᵢ" is the initial impedance, and "ΔZₙ" is the expected change in impedance.

In one aspect, a method of determining efficacy of a pulsed electric field (PEF) ablation procedure is provided. The method includes generating at least one pulsed electric field (PEF) pulse to be delivered to at least one electrode of a plurality of electrodes, the at least one electrode being at a distal end of a PEF ablation catheter and being positionable in proximity to a target region of tissue to be ablated. The method also includes determining an index of completeness indicative of a completeness of ablation of the target region of tissue based at least in part on a change in a parameter compared to an expected change in the parameter, the change in the parameter being caused at least in part on an extent of ablation of the target region.

In some embodiments, the index of completeness is a ratio of the achieved depth, size, or width during delivery of PEF energy to the target tissue versus a predetermined depth, size, or width of lesion formation.

In some embodiments, the method further includes terminating the delivery of PEF energy to the medical device if a threshold for an acceptable ratio of completeness is achieved.

In some embodiments, the method further includes generating an alert if the determined ratio of completeness exceeds a predetermined acceptable ratio of completeness threshold.

In some embodiments, the method further includes displaying the index of completeness for at least one of: each electrode of the plurality of electrodes, a subset of the plurality of electrodes and a totality of the electrodes.

In some embodiments, displaying the index of completeness includes displaying an area about the plurality of electrodes treated with PEF energy.

In some embodiments, displaying the index of completeness includes displaying a color-coded indicator of a magnitude of the index of completeness for each of the plurality of electrodes.

In some embodiments, the measuring includes measuring impedance for each of at least one pair of electrodes.

In some embodiments, the measuring includes measuring the temperature of the plurality of electrodes.

In some embodiments, the index of completeness is a measure including at least one from the group consisting of depth, volume and width of the lesion created.

In one aspect, a processing circuitry for a medical system includes processing circuitry configured to measure temperature or an impedance of at least one of a plurality of electrodes of a medical device coupled to the processing circuitry during delivery of pulsed electric field (PEF) energy from the plurality of electrodes to a target tissue to create a lesion. A ratio of completeness of the created lesion is determined based at least in part on the measured temperature or the impedance. The delivery of PEF energy to the target tissue is terminated if a threshold for an acceptable ratio of completeness is achieved.

In some embodiments, the index of completeness is determined from a non-linear function of at least one of a temperature, electrogram, voltage amplitude, delivered current and an electrode impedance.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF THE DRAWINGS

A more complete understanding of the present invention, and the attendant advantages and features thereof, will be more readily understood by reference to the following detailed description when considered in conjunction with the accompanying drawings wherein:
FIG. 1 is a system view of an exemplary pulsed electric field (PEF) energy delivery system constructed in accordance with the principles of the present application;
FIG. 2 is a flow chart of an exemplary method of determining an index of completeness of a PEF ablation;
FIG. 3 is a flowchart of another example process for determining an index of completeness of a PEF ablation;
FIG. 4 is a side view of an exemplary graphical display of electrodes of the medical device shown in FIG. 1 showing an impedance between electrodes between adjacent electrodes;
FIG. 5 is a side view of an exemplary graphical display of electrodes of the medical device shown in FIG. 1 showing bars above each electrode indicating a percentage of completeness of an ablation associated with the electrode; and
FIG. 6 is a top view of an exemplary graphical display of electrodes and a percentage of completeness of an ablation about the circumference of the electrodes.

### DETAILED DESCRIPTION

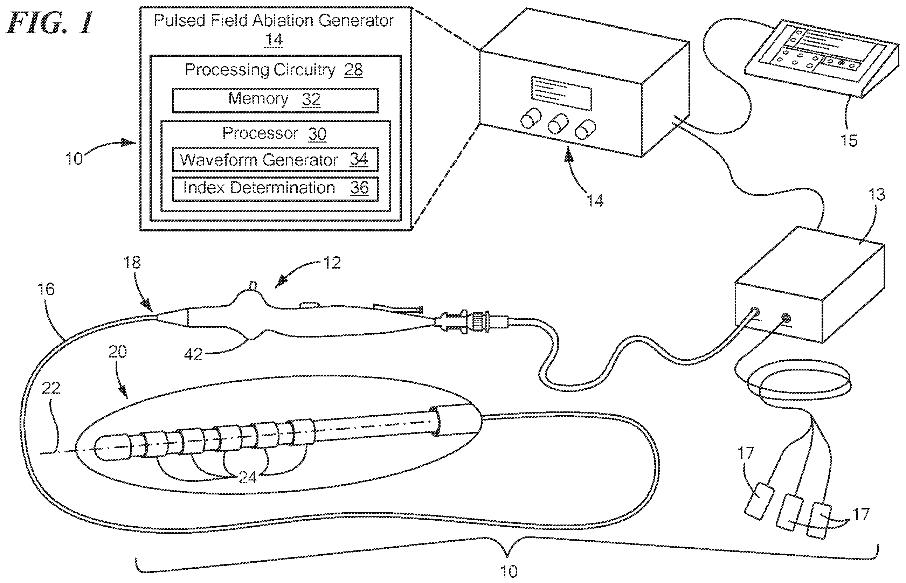

FIG. 1 shows an example embodiments of a medical system 10 configured to determine a ratio of completeness of a lesion obtained by application of pulsed electric field (PEF) pulses using a medical device 12 that has a pulsed field ablation generator 14 contained in a controller 15, as shown in FIG. 1. In some embodiments, the pulsed field ablation generator 14 is configured to determine one or more indices of completeness, where an index of completeness may be based on one or more measured or calculated parameters such as temperature, impedance, voltage, current, power, and EGM, for example. Some purposes of some embodiments herein disclosed include providing a clinician with a visual indication of a determined measure of completeness of the lesion so that the clinician can make an informed judgement about whether to apply additional therapy and what that additional therapy should be. Further details are disclosed below.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

As used herein, a "ratio of completeness" is a numerical value based on a ratio of a measured or calculated parameter to an expected parameter, or on a ratio of a change in the measured or calculated parameter over a time interval to an expected or predicted change in that parameter over the same time interval. As used herein, an "index of completeness" is a numerical value based on one or more ratios of completeness, such as a weighted sum of ratios of completeness, for example. Thus, when the index of completeness is based on only one ratio of completeness, that ratio of completeness may be referred to as an index of completeness. In general, the parameters that contribute to a ratio of completeness or an index of completeness include tissue characteristics associated with a lesion such as depth, volume, width, transmurality or continuity of the lesion creation, etc., as described in greater detail below.

Referring now to the drawing figures in which like reference designations refer to like elements, an embodiment of the medical system 10 constructed in accordance with principles disclosed herein is shown in FIG. 1 The medical system 10 generally includes the medical device 12 that may be coupled directly to the pulsed field ablation generator 14. The pulsed field ablation generator provides control of the delivery and monitoring of pulsed electric field (PEF) pulses through a catheter electrode distribution system 13. In some embodiments, the catheter electrode distribution system 13 may be included within the pulsed field ablation generator 14. A controller 15 may further be included in communication with the generator for operating and controlling the various functions of the generator 14 and in further communication with one or more surface electrodes 17 configured to measure and record electrograms.

The medical device 12 may generally include one or more diagnostic or treatment regions for energetic, therapeutic and/or investigatory interaction between the medical device 12 and a treatment site. The treatment region(s) may deliver, for example, pulsed electric field (PEF) energy sufficient to reversibly or irreversibly electroporate a tissue area, or radio frequency energy in proximity to the treatment region(s).

The controller 15 may include a video display and/or keyboard and/or a mouse and may be connected to the pulsed field ablation generator 13 wirelessly, optically or by wire. The controller 15 may be an application program on a handheld, laptop, tablet or desktop device, such as a wireless smart phone or a desktop computer, for example. Thus, results of determinations of ratios and indices of completeness may be stored, displayed and transported electronically to another location or another device.

The medical device 12 may include an elongate body or catheter 16 passable through a patient's vasculature and/or positionable proximate to a tissue region for diagnosis or treatment, such as a catheter, sheath, or intravascular introducer. The elongate body, shaft, or catheter 16 may define a proximal portion 18 and a distal portion 20, and may further include one or more lumens disposed within the elongate body 16 thereby providing mechanical, electrical, and/or fluid communication between the proximal portion of the elongate body 16 and the distal portion of the elongate body 16. The distal portion 20 may generally define the one or more treatment region(s) of the medical device 12 hat are operable to monitor, diagnose, and/or treat a portion of a patient.

The treatment region(s) may have a variety of configurations to facilitate such operation. In the case of purely bipolar pulsed field delivery, distal portion 20 includes electrodes that form the bipolar configuration for energy delivery. The electrodes 20 may be in a linear configuration along an axis 22 or in a non-linear (curved) configuration. For example, the electrodes may be linear at a time of inserting the distal portion into proximity of the tissue to be ablated, and then subsequently expanded into a non-linear configuration. Further, in some embodiments, a plurality of active electrodes 24 may serve as one pole while a second device containing one or more electrodes may be placed to serve as the opposing pole of the bipolar configuration.

The pulsed field ablation generator 14 includes processing circuitry 28 for control, delivery and monitoring of a pulsed field waveform to one or more of the active electrodes 24. The processing circuitry may be implemented by a processor 30 in communication with a memory 32. The memory 32 is configured to store measurements that may come from one or more of the active electrodes 24, one or more other electrodes and/or sensors, such as temperature sensors. The memory 32 is also configured to store numerical values that are derived from, or based at least in part on, the measurements from one or more sensors and/or electrodes.

The processor 30 may include a waveform generator 34 which is configured to generate electrical pulses that may include multiple pulses delivered in a pattern or randomly. The waveform generator may be in communication with the active electrodes 24 directly or indirectly in order to deliver pulses of energy to the active electrodes 24. The processor 30 includes an index determination unit 36 which is configured to determine, based on some or all of the measurements, and/or based on predicted or experimental values, at least one index indicative of a completeness of the lesion. The measurements may include one or more of temperature, voltage amplitude, current, and impedance.

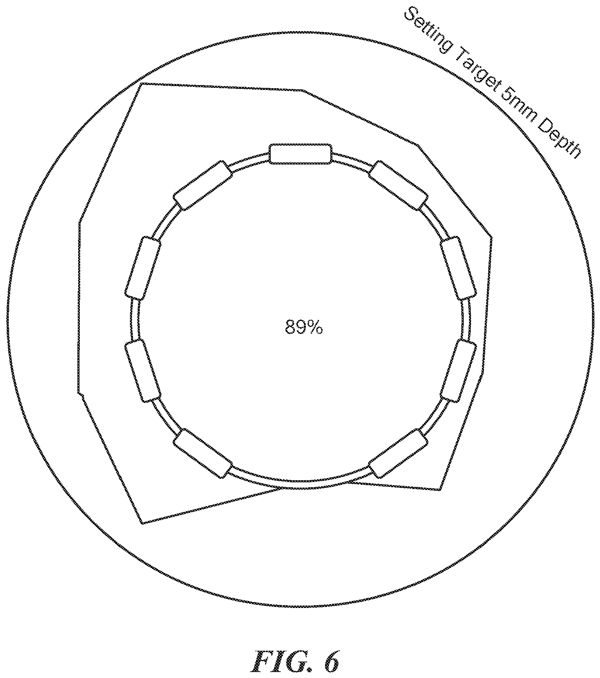

The active electrodes 24 may be in a linear configuration or in a configuration having curvature. For example, the distal portion 20 may include six active electrodes 24 linearly disposed along a common longitudinal axis 22, as shown in FIG. 1. Alternatively, the distal portion 20 may include an electrode carrier arm or splines that are transitionable between a linear configuration and an expanded configuration in which the carrier arm or splines have an arcuate or substantially circular or elliptical configuration, for example. The carrier arm or splines may include the plurality of active electrodes 24 that are configured to deliver PEF energy. Further, the carrier arm when in the expanded configuration may lie in a plane that is substantially orthogonal to the longitudinal axis of the elongate body 16. (See, for example, FIG. 6.) The planar orientation of the expanded carrier arm may facilitate ease of placement of the plurality of active electrodes 24 in contact with the target tissue.

In particular, in addition to or instead of a processor 30, such as a central processing unit, and memory, the processing circuitry 28 may comprise integrated circuitry for processing and/or control, e.g., one or more processors and/or processor cores and/or FPGAs (Field Programmable Gate Array) and/or ASICs (Application Specific Integrated Circuitry) adapted to execute instructions. The processor 30 may be configured to access (e.g., write to and/or read from) the memory 32, which may comprise any kind of volatile and/or nonvolatile memory, e.g., cache and/or buffer memory and/or RAM (Random Access Memory) and/or ROM (Read-Only Memory) and/or optical memory and/or EPROM (Erasable Programmable Read-Only Memory).

Thus, the memory 32 is configured to store software and/or data. Some data and software may be retrievable from external memory (e.g., database, storage array, network storage device, etc.) accessible by the pulsed field ablation generator 14 via an external connection. Software may be executable by the processing circuitry 28. The processing circuitry 28 may be configured to control any of the methods and/or processes described herein and/or to cause such methods, and/or processes to be performed, e.g., by the pulsed field ablation generator 14. Processor 30 corresponds to one or more processors 30 for performing pulsed field ablation generator functions described herein. The memory 32 is configured to store data, programmatic software code and/or other information described herein. In some embodiments, the software stored in the memory 32 may include instructions that, when executed by the processor 30 and/or processing circuitry 28, causes the processor 30 and/or processing circuitry 28 to perform the processes described herein with respect to the pulsed field ablation generator 14.

Details for determining the index indicative of a completeness of a lesion are provided below. In some embodiments, the index indicative of a completeness of the lesion may be based on a ratio of two quantities and be referred to herein as an index of completeness. Some purposes of determining a ratio of two measured or calculated parameters is to provide an index that is proportional to how much larger or smaller the actual change in the parameter over a time interval is compared to an expected or predicted change in the parameter over that same time interval. In this way, the techniques described herein can be used to provide real-time feedback regarding lesion formation. In some examples, a clinician may use the feedback to determine a desired subsequent ablation therapy.

In an example for purposes of illustration, a ratio of completeness may be determined based on a transmurality parameter. In this example, the index determiner 36 may determine an expected transmurality based on lesion depth or tissue transmurality. Tissue thickness may be measured from imaging techniques such as magnetic resonance imaging (MRI), CT, Echo, etc., or may be based on measured tissue properties such as impedance.

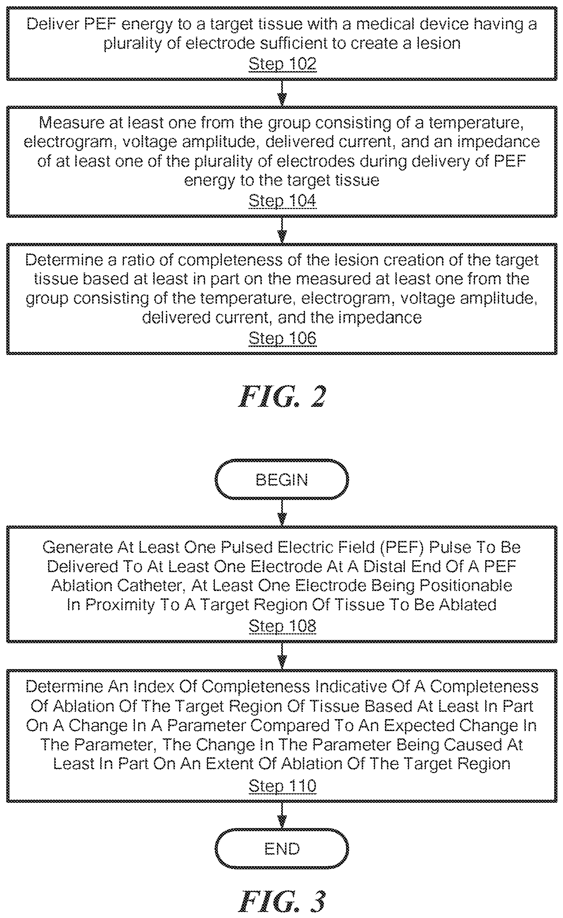

FIG. 2 is a flowchart of one example process for determining efficacy of a PEF ablation procedure utilizing a ratio of index of completeness. In particular, the method includes delivering PEF energy to a target tissue with the medical device 12 having the plurality of electrodes 24 sufficient to create a lesion (Step 100). In one configuration, the plurality of electrodes 24 is placed in direct contact with the target tissue, and in other configurations, the plurality of electrodes 24 is placed proximate to the tissue to be treated. At least one of a temperature, voltage amplitude, delivered current, and impedance (e.g., therapy delivery parameters) of at least one of the plurality of electrodes 24 is measured during delivery of PEF energy to the target tissue (Step 102).

Optionally, EGM measurements may be taken from the surface electrodes 17 in communication with the controller 15 proximate in time to the delivery of PEF energy to the tissue. In particular, during the delivery of PEF energy to the target tissue, the controller 15 is configured to measure the temperature, voltage amplitude, delivered current, and/or impedance of each of the plurality of electrodes 24, between adjacent ones of the plurality of electrodes 24, and/or between one of the plurality of electrodes 24 and a ground electrode (not shown). For example, if there are 5 electrodes, each electrode 24 may include a thermocouple (not shown) configured to measure the temperature of each of the 5 electrodes as PEF energy is delivered to tissue.

A ratio or index of completeness of the lesion created based at least in part on the measured at least one of the temperature, voltage amplitude, delivered current, and impedance is then determined (Step 104). The index of completeness may be the ratio of computed quantities based on measured parameter(s) The measured parameters may be associated with either the desired change in tissue property and/or the successful delivery of therapeutic energy. For example, a measurement of the impedance before and after the delivery of ablative therapy may indicate whether the tissue has been affected in the desired way (such as the target tissue impedance change being achieved). For example, a measure of the temperature rise following or persisting after a delivery of energy may indicated whether the electrodes were in contact with tissue (indicating the energy was delivered where desired).

For example, a ratio of completeness for each electrode 24 may be determined based at least in part on calculating the following equation: $C = k \times \frac{\left({T}_{f}-{T}_{i}\right)}{Δ {T}_{n}}$, where "C" is a ratio of completeness, "k" is a constant, "T_{f}" is a final temperature measured at an end of a time interval, "Tᵢ" is the initial temperature measured at a beginning of the time interval, and "Tₙ" is the expected or predicted change in temperature over that time interval. The expected or predicted change may be derived from modeling or empirical observation. The expected or predicted values and model parameters for determining the expected or predicted values may be stored in the memory 32.

Alternatively, or additionally, a ratio of completeness may be calculated by the following equation: $C = k \times \frac{\left({Z}_{f}-{Z}_{i}\right)}{Δ {Z}_{n}}$, where "C" is a ratio of completeness, "k" is a constant and "Z_{f}" is the final impedance at the end of the time interval, "Zᵢ" is the initial impedance at the beginning of the time interval, and "Zₙ" is the expected or predicted change in impedance over that time interval. The expected or predicted change that may be derived from modeling or empirical observation. Voltage amplitude, delivered current, and EGM measurement assessments of completeness may be calculated in the same or similar manner.

FIG. 3 is a flowchart of another example process performable by the pulsed field ablation generator 14, processing circuitry 28, waveform generator 34 and index determination unit 36. The process includes generating at least one pulsed electric field (PEF) pulse to be delivered to at least one electrode at a distal end of a PEF ablation catheter, at least one electrode 24 being positionable in proximity to a target region of tissue to be ablated (Step 110). The process also includes determining an index of completeness indicative of a completeness of ablation of the target region of tissue based at least in part on a change in a parameter compared to an expected change in the parameter, the change in the parameter being caused at least in part on an extent of ablation of the target region (Step 112).

In some embodiments, the index of completeness is based at least in part on a ratio of the achieved depth, size, or width from delivery of PEF energy to the target tissue versus a predetermined depth, size, or width of lesion formation (e.g., lesion parameters).

In some embodiments, the process includes terminating the delivery of PEF energy to the medical device 12 if a threshold for an acceptable ratio of completeness is achieved. For example, X stores a threshold. Example thresholds are... In some embodiments, the process also includes generating an alert if the determined ratio of completeness exceeds a predetermined acceptable ratio of completeness threshold.

In some embodiments, the method also includes displaying the index of completeness for at least one of: each of the plurality of electrodes, a subset of the plurality of electrodes and a totality of the electrodes. In some embodiments, displaying the index of completeness includes displaying an area about the plurality of electrodes 24 treated with PEF energy. In some embodiments, displaying the index of completeness includes displaying a color-coded indicator of a magnitude of the index of completeness for each of the plurality of electrodes. In some embodiments, the method also includes measuring impedance for each of at least one pair of electrodes.

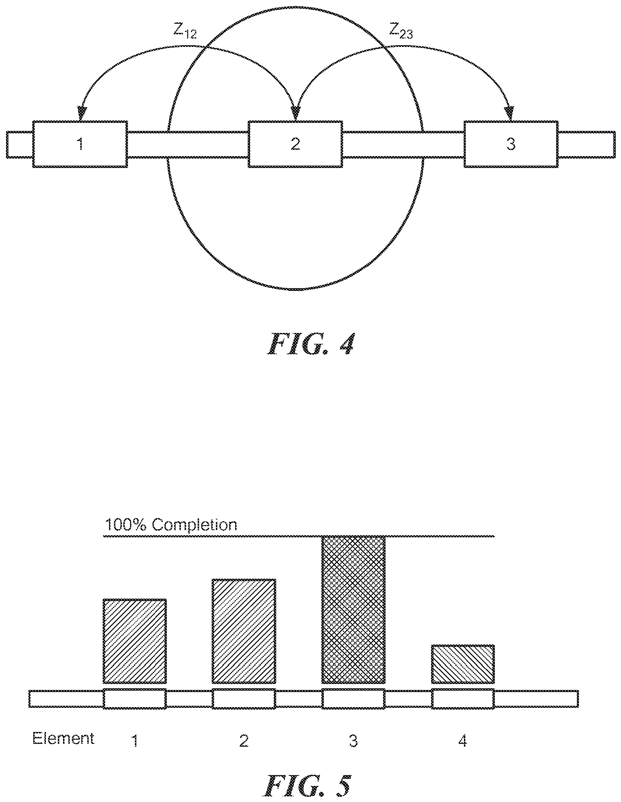

The impedance may be measured at each electrode 24, between electrodes 24, or between an electrode 24 and a ground electrode. For example, in the case of bipolar impedance between electrodes 1, 2, and 3, as shown in FIG. 4, the measurements may have multiple paths of assessment. For example, in the case of the three electrodes shown in FIG. 4, which may be a subset of a larger number of electrodes, the processing circuitry 28 and index determination unit 36 may determine the following individual impedance ratios: ${C}_{12} = {k}_{12} * \left({Z}_{12 f}-{Z}_{12 b}\right) / {dZ}_{12}$ ${C}_{23} = {k}_{23} * \left({Z}_{23 f}-{Z}_{23 b}\right) / {dZ}_{23}$
where f denotes a final measured impedance, k is a factor which may be determined experimentally and retrieved from memory, the Z₁₂ is the impedance presented between electrodes 1 and 2, dZ₁₂ is the expected difference in impedance between electrodes 1 and 2, Z₂₃ is the impedance presented between electrodes 2 and 3, dZ₂₃ is the expected difference in impedance between electrodes 2 and 3. These individual impedance ratios may be combined as follows: ${C}_{Total} = {Σω}_{i} {C}_{i}$
where "ω" is a weight and the sum is over a number of pairs of electrodes. The weights ωi may be based on, for example, the conductivity of the electrode materials or the expected fidelity of a particular measurement of the electrodes 24 and tissue targets. Note that ωi may be incorporated into constant "kᵢ." The constants "kᵢ" or weights "ωᵢ" may be further dependent on the initial measurement values for example Zᵢ or Tᵢ, and indices of completeness may be combined from multiple forms using C_{Total} = ΣωᵢCᵢ, for example. Other methods of combining different measured or calculated parameters may also be used, such as a product of the form, C_{Total} = ΠωᵢCᵢ. In some embodiments, a Kalman filtering method may be employed to determine the index of completeness.

Therefore, in some examples, an index of completeness may be based on one or more measured or calculated parameters, such as expected temperature rise, for example. For example, several ratios of completeness may be combined based on the combined ratios of completeness for each electrode 24, the controller 15 is configured to display on a display the ratio of completeness for at least one of: each of the plurality of electrodes, a subset of the plurality of electrodes and a totality of the electrodes 24. The ratio of completeness may be displayed in a variety of ways, such as a color pattern, bar graph or line graph displaying the ratio versus a parameter such as impedance or temperature. For example, a display of a bar or circumferential array constructed from similar data to suggest completion between electrodes compared to thresholds. The measurements may indicate a super-treatment threshold. The display may indicate a response to ablative therapy that is above or below a threshold. alerts/alarms/visualizations (i.e. a separate alert bar reiterates or reads out the note that delivery has surpassed the value, presumably by some defined threshold, i.e. 5% excess may be within margin of error depending on the values used in the computation, but a 100% difference may suggest excessive heat is applied if measuring temperature, for example.

**In** some embodiments, an index of completeness can be determined based on one or more ratios, each ratio being itself based on a different one of the measured or calculated parameters. For example, one value of C₁ can be based on a ratio of impedances or impedance differences and another value of C₂ can be based on a ratio of temperatures or temperature differences and yet another value C₃ can be based on a ratio of voltages or voltage differences, and so on. Each of these values of Cᵢ based on different measured or calculated parameters can be combined according to a nonlinear or linear function.

For example, an index of completeness may be a weighted sum of these values of Cᵢ. Purposes for computing a combination of Cᵢ based on different measured and/or calculated parameters include enabling a graphical display of a two or three dimensional view on a video monitor. For example, in some embodiments, a first display may present a three-dimensional plot of the magnitude of the ratio of completeness versus temperature and versus impedance. This would enable the clinician to make a judgement about what temperature and impedance combination to expect for a desired ratio of completeness, such as at least 80%, for example. In some embodiments, combinations of temperature and impedance values may be mapped by the processing circuitry 28 to voltages and currents to be applied to various electrodes by the catheter electrode distribution system 13 under the control of the processing circuitry 28.

For example, each electrode 24 of the plurality of electrodes 24 may be displayed linearly, as shown in FIG. 5, and the magnitude of a ratio of completeness for each electrode may be shown by a bar having a height that is proportional to the magnitude of the ratio of completeness for that electrode. Other visual representations are possible, such as showing a percentage or color-coded display based on either the measured temperature, voltage amplitude, delivered current, and/or impedance, or other measured or calculated parameter. For example, a bar or graph may be displayed in a discrete or continuous set of colors and/or degrees of opacity, where the color or degree of opacity depends on the magnitude of the ratio of completeness of an electrode or an index of completeness determined from one or more of the values C_{i,j}, referenced above. Some purposes of displaying a magnitude of one or more ratios or indices of completeness by a color gradient or degree of opacity may include enabling the clinician to instantly see what electrodes and/or measured/calculated parameters have a greatest effect upon the ratio or index of completeness.

Optionally, the controller 15 may be configured to display an area or volume of space and/or tissue surrounding one or more of the plurality of electrodes 24 treated with PEF energy. An intensity, color, graph and/or degree of opacity may be used to display a measured and/or calculated parameter and/or one or more ratios and/or indices of completeness. For example, in the case of a circumferential lesion, with a predetermined, predicted, or specified depth, volume, width, transmurality or continuity of lesion creation, the controller 15 may generate an image about the plurality of electrodes 24 illustrating the depth, volume, width, transmurality or continuity of lesion creation achieved as well as one or more of ratios and/or or indices of completeness for each of the individual electrodes 24. In addition to displaying the achieved lesion parameters, such as depth, volume, width, transmurality or continuity, the controller 15 may display the predetermined, predicted or specified lesion parameters as an overlay of the displayed achieved lesion parameters.

Thus, in one configuration, the controller 15 is configured to be set or programmed with a predetermined depth, width, volume, transmurality, or continuity of lesion formation for given parameters of a PEF application. For example, as shown in FIG. 6, a target predetermined lesion depth of 5mm may be programmed by the clinician or preset by the pulsed field ablation generator 14and the ratio of completeness may be a ratio of the achieved depth during delivery of PEF energy to the target tissue versus the predetermined depth of lesion formation. As an example, the processing circuitry 28 may be configured to predict a ratio or index of completeness for a particular combination of therapeutic energy applied to a group of electrodes. In some embodiments, the processing circuitry 28 may be configured to compare the predicted ratio or index to that achieved by an application of the combination of voltages to the group of electrodes. In some embodiments, the processing circuitry 28 may be configured to predict a lesion depth for a particular combination of voltages applied to a group of electrodes based on a display by the controller 15 of one or more ratios or indices of completeness versus lesion depth. For example, suppose an achieved lesion depth, is 4mm. This may be divided by a predetermined or predicted depth of lesion formation, for example, 5mm to arrive at an index of completeness of 80% or 0.80. This may be determined experimentally to correlate with changes in measured temperature and/or changes in impedance, for example, over an interval of time. An index or ratio of completeness may be therefore expressed in terms of a "percentage complete."

A ratio of completeness may be determined for each of a plurality of electrodes 24 based on a non-linear or linear function of the measured variable or variables. For example, the ratio of completeness may be determined as a non-linear function of temperature, electrogram(s), applied voltage amplitude, applied current amplitude and/or impedance between electrodes 24 and an acceptable ratio of completeness.

Accordingly, the pulsed field ablation generator 14 may be configured with a threshold for an acceptable ratio of completeness. Then, the controller may be configured to terminate generation of PEF energy to the medical device 12 if the threshold for an acceptable ratio of completeness is achieved. Additionally, or alternatively, the pulsed field ablation generator 14 may be configured to generate an alert if the determined ratio of completeness exceeds a predetermined acceptable ratio of completeness threshold. For example, if 80% is the desired ratio of completeness, the pulsed field ablation generator 14may terminate the delivery of PEF energy or generate an alert when the 80% threshold is reached.

For a given application of pulsed electric field pulses, a target value of completeness is unachievable, such as if a catheter were to move from the original target location during ablation. In such a case, the pulsed field ablation generator 14 may be configured to cause discontinuation or change in the delivery of therapy or sets of therapy. Discontinuation or change in therapy may also automatically or manually be triggered upon detecting a minimal or negative change or successive changes in the completeness ratio with each successive application of ablative therapy (i.e., with each successive application of pulsed electric field pulses to the electrodes 24).

For example, if a catheter were to move from a position of tissue contact to a free-floating position in blood, the expected temperature change associated with successful delivery of ablative therapy during a time interval may a small positive number. In such case, when an actual measured temperature change is repeatedly less than expected or opposite in direction from the expected direction of temperature change, indicating increased cooling from blood, an alarm, indicator, or display may be generated by the controller 15 under direction by the pulsed field ablation generator 14 to indicate that a desired ratio or index of completion is not being achieved. Once again, the pulsed field ablation generator 14 may be configured to cause discontinuation or change in the delivery of the ineffectual therapy.

Certain techniques of this disclosure are set forth in the following clauses.

Clause 1: A method in a medical device of determining efficacy of a pulsed electric field (PEF) ablation procedure, the method comprising: generating at least one pulsed electric field (PEF) pulse to be delivered to at least one electrode of a plurality of electrodes, the at least one electrode being at a distal end of a PEF ablation catheter and being positionable in proximity to a target region of tissue to be ablated; and measure at least one parameter; determining an index of completeness indicative of a completeness of ablation of the target region of tissue, the index of completeness determined based at least in part on a change in a measured parameter relative to an expected change in the measured parameter, the change in the measured parameter being caused at least in part on an extent of ablation of the target region.

Clause 2: The method of clause 1, wherein the index of completeness is based at least in part on a ratio of an achieved depth, size, or width from delivery of PEF energy to the target tissue versus a predetermined depth, size, or width of lesion formation.

Clause 3: The method of clause 1 or 2, further comprising terminating delivery of PEF energy to the medical device based on achieving a threshold for an acceptable ratio of completeness.

Clause 4: The method of any of clauses 1-3, further comprising generating an alert based on the determined ratio of completeness exceeds a predetermined acceptable ratio of completeness threshold.

Clause 5: The method of any of clauses 1-4, further comprising displaying the index of completeness for at least one of the plurality of electrodes.

Clause 6: The method of clause 5, wherein displaying the index of completeness includes displaying an area about the plurality of electrodes treated with PEF energy.

Clause 7: The method of clause 5 or 6, wherein displaying the index of completeness includes displaying a color-coded indicator of a magnitude of the index of completeness for each of the plurality of electrodes.

Clause 8: The method of any of clauses 1-7, further comprising measuring impedance for each of at least one pair of electrodes of the plurality of electrodes.

Clause 9: The method of any of clauses 1-8, wherein determining the index of completeness includes determining $C = k \times \frac{\left({T}_{f}-{T}_{i}\right)}{Δ {T}_{n}}$, where "k" is a constant, *"T_{f}"* is the final temperature, "*Tᵢ*" is the initial temperature, and "Δ*Tₙ"* is the expected change in temperature.

Clause 10: The method of any of clauses 1-9, wherein determining the index of completeness includes determining $C = k \times \frac{\left({Z}_{f}-{Z}_{i}\right)}{Δ {Z}_{n}}$, where "k" is a constant and "Z_{f}" is the final impedance, "Zᵢ" is the initial impedance, and "ΔZₙ" is the expected change in impedance.

As will be appreciated by one of skill in the art, the concepts described herein may be embodied as a method, data processing system, computer program product and/or computer storage media storing an executable computer program. Accordingly, the concepts described herein may take the form of an entirely hardware embodiment, an entirely software embodiment or an embodiment combining software and hardware aspects all generally referred to herein as a "circuit" or "module." Any process, step, action and/or functionality described herein may be performed by, and/or associated to, a corresponding module, which may be implemented in software and/or firmware and/or hardware. Furthermore, the disclosure may take the form of a computer program product on a tangible computer usable storage medium having computer program code embodied in the medium that can be executed by a computer. Any suitable tangible computer readable medium may be utilized including hard disks, CD-ROMs, electronic storage devices, optical storage devices, or magnetic storage devices.

Some embodiments are described herein with reference to flowchart illustrations and/or block diagrams of methods, systems and computer program products. It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer (to thereby create a special purpose computer), special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable memory or storage medium that can direct a computer or other programmable data processing apparatus to function in a particular manner, such that the instructions stored in the computer readable memory produce an article of manufacture including instruction means which implement the function/act specified in the flowchart and/or block diagram block or blocks.

The computer program instructions may also be loaded onto a computer or other programmable data processing apparatus to cause a series of operational steps to be performed on the computer or other programmable apparatus to produce a computer implemented process such that the instructions which execute on the computer or other programmable apparatus provide steps for implementing the functions/acts specified in the flowchart and/or block diagram block or blocks.

It is to be understood that the functions/acts noted in the blocks may occur out of the order noted in the operational illustrations. For example, two blocks shown in succession may in fact be executed substantially concurrently or the blocks may sometimes be executed in the reverse order, depending upon the functionality/acts involved. Although some of the diagrams include arrows on communication paths to show a primary direction of communication, it is to be understood that communication may occur in the opposite direction to the depicted arrows.

Computer program code for carrying out operations of the concepts described herein may be written in an object oriented programming language such as Python, Java^{®} or C++. However, the computer program code for carrying out operations of the disclosure may also be written in conventional procedural programming languages, such as the "C" programming language. The program code may execute entirely on the user's computer, partly on the user's computer, as a stand-alone software package, partly on the user's computer and partly on a remote computer or entirely on the remote computer. In the latter scenario, the remote computer may be connected to the user's computer through a local area network (LAN) or a wide area network (WAN), or the connection may be made to an external computer (for example, through the Internet using an Internet Service Provider).

Many different embodiments have been disclosed herein, in connection with the above description and the drawings. It will be understood that it would be unduly repetitious and obfuscating to literally describe and illustrate every combination and subcombination of these embodiments. Accordingly, all embodiments can be combined in any way and/or combination, and the present specification, including the drawings, shall be construed to constitute a complete written description of all combinations and subcombinations of the embodiments described herein, and of the manner and process of making and using them, and shall support claims to any such combination or subcombination.

It will be appreciated by persons skilled in the art that the present invention is not limited to what has been particularly shown and described herein above. In addition, unless mention was made above to the contrary, it should be noted that all of the accompanying drawings are not to scale. A variety of modifications and variations are possible in light of the above teachings without departing from the scope of the following claims.

## Claims

1. A medical system (10), comprising:
a generator (14) comprising processing circuitry (28) configured to:
generate at least one pulsed electric field, PEF, pulse to be delivered to at least one electrode (24) of a plurality of electrodes (24), the at least one electrode (24) being at a distal end (20) of a PEF ablation catheter (16) and being positionable in proximity to a target region of tissue to be ablated;
store measurements of parameters; and **characterised in that** said generator is further configured to
determine an index of completeness indicative of a completeness of ablation of the target region of tissue, the index of completeness determined based at least in part on a change in a measured parameter relative to an expected change in the measured parameter, the change in the measured parameter being caused at least in part on an extent of ablation of the target region.

2. The system (10) of Claim 1, wherein the processing circuitry (28) is configured to set a predetermined depth, size, or width of lesion formation, and wherein the index of completeness comprises a ratio of an achieved depth, size, or width from delivery of PEF energy to the target tissue versus the predetermined depth, size, or width of lesion formation.

3. The system (10) of Claim 1 or 2, wherein the processing circuitry (28) is configured to be preprogrammed with a threshold for an acceptable ratio of completeness, and wherein the processing circuitry (28) is configured terminate the generation of PEF energy to the medical device based on achieving the threshold for the acceptable ratio of completeness.

4. The system (10) of any of Claims 1-3, wherein the processing circuitry (28) is configured to generate an alert based on the determined ratio of completeness exceeding a predetermined acceptable ratio of completeness threshold.

5. The system (10) of any of Claims 1-4, further comprising a display in communication with the processing circuitry (28) and the medical device, and wherein the processing circuitry (28) is configured to cause display of the index of completeness for at least a subset of the plurality of electrodes (24).

6. The system (10) of Claim 5, wherein the processing circuitry (28) is configured to cause display of an area about the plurality of electrodes (24) treated with PEF energy.

7. The system (10) of any of Claims 1-6, wherein the processing circuitry (28) is configured to cause display of at least one of a color-coded indicator and a variable opacity based on the ratio of completeness for each of the plurality of electrodes (24).

8. The system (10) of any of Claims 1-7, wherein the processing circuitry (28) is configured to measure impedance of at least one of each electrode (24), between adjacent electrodes (24), between one of the plurality of electrodes (24) and a ground electrode (24).

9. The system (10) of any of Claims 1-8, wherein determining the index of completeness comprises determining $C = k \times \frac{\left({T}_{f}-{T}_{i}\right)}{Δ {T}_{n}}$, where "k" is a constant, *"T_{f}"* is the final temperature, "*Tᵢ*" is the initial temperature, and "Δ*Tₙ"* is the expected change in temperature.

10. The system (10) of any of Claims 1-9, wherein determining the index of completeness comprises determining $C = k \times \frac{\left({Z}_{f}-{Z}_{i}\right)}{Δ {Z}_{n}}$, where "k" is a constant and "Z_{f}" is the final impedance, "Zᵢ" is the initial impedance, and "ΔZₙ" is the expected change in impedance.

## Patentansprüche

1. Medizinisches System (10), das Folgendes umfasst:
einen Generator (14), der eine Verarbeitungsschaltungsanordnung (28) umfasst, die zu Folgendem konfiguriert ist:
Erzeugen mindestens eines Impulses eines gepulsten elektrischen Felds, PEF-Impuls, der an mindestens eine Elektrode (24) mehrerer Elektroden (24) abzugeben ist, wobei sich die mindestens eine Elektrode (24) an einem distalen Ende (20) eines PEF-Ablationskatheters (16) befindet und in der Nähe einer Zielregion von zu abladierendem Gewebe positionierbar ist;
Speichern von Messwerten von Parametern; und **dadurch gekennzeichnet, dass** der Generator ferner dazu konfiguriert ist, einen Vollständigkeitsindex, der eine Vollständigkeit der Ablation der Zielregion von Gewebe angibt, zu bestimmen, wobei der Vollständigkeitsindex zumindest teilweise auf einer Änderung eines gemessenen Parameters relativ zu einer erwarteten Änderung des gemessenen Parameters basiert, wobei die Änderung des gemessenen Parameters zumindest teilweise durch ein Ausmaß der Ablation der Zielregion verursacht wird.

2. System (10) nach Anspruch 1, wobei die Verarbeitungsschaltungsanordnung (28) dazu konfiguriert ist, eine vorgegebene Tiefe, Größe oder Breite einer Läsionsbildung einzustellen, und wobei der Vollständigkeitsindex ein Verhältnis einer erreichten Tiefe, Größe oder Breite aus der Abgabe von PEF-Energie an das Zielgewebe zu der vorgegebenen Tiefe, Größe oder Breite der Läsionsbildung umfasst.

3. System (10) nach Anspruch 1 oder 2, wobei die Verarbeitungsschaltungsanordnung (28) dazu konfiguriert ist, mit einem Schwellenwert für ein akzeptables Vollständigkeitsverhältnis vorprogrammiert zu werden, und wobei die Verarbeitungsschaltungsanordnung (28) dazu konfiguriert ist, die Erzeugung von PEF-Energie an die medizinische Vorrichtung basierend auf dem Erreichen des Schwellenwerts für das akzeptable Vollständigkeitsverhältnis zu beenden.

4. System (10) nach einem der Ansprüche 1-3, wobei die Verarbeitungsschaltungsanordnung (28) dazu konfiguriert ist, einen Alarm basierend darauf zu erzeugen, dass das bestimmte Vollständigkeitsverhältnis einen vorgegebenen Schwellenwert für ein akzeptables Vollständigkeitsverhältnis überschreitet.

5. System (10) nach einem der Ansprüche 1-4, das ferner eine Anzeige in Kommunikation mit der Verarbeitungsschaltungsanordnung (28) und der medizinischen Vorrichtung umfasst, wobei die Verarbeitungsschaltungsanordnung (28) dazu konfiguriert ist, eine Anzeige des Vollständigkeitsindexes für mindestens eine Teilmenge der mehreren Elektroden (24) zu bewirken.

6. System (10) nach Anspruch 5, wobei die Verarbeitungsschaltungsanordnung (28) dazu konfiguriert ist, eine Anzeige eines mit PEF-Energie behandelten Bereichs um die mehreren Elektroden (24) herum zu bewirken.

7. System (10) nach einem der Ansprüche 1-6, wobei die Verarbeitungsschaltungsanordnung (28) dazu konfiguriert ist, eine Anzeige eines farbcodierten Indikators und/oder einer variablen Opazität basierend auf dem Vollständigkeitsverhältnis für jede der mehreren Elektroden (24) zu bewirken.

8. System (10) nach einem der Ansprüche 1-7, wobei die Verarbeitungsschaltungsanordnung (28) dazu konfiguriert ist, die Impedanz mindestens einer von jeder Elektrode (24), zwischen benachbarten Elektroden (24), zwischen einer der mehreren Elektroden (24) und einer Masseelektrode (24) zu messen.

9. System (10) nach einem der Ansprüche 1-8, wobei das Bestimmen des Vollständigkeitsindexes das Bestimmen von $C = k x \frac{\left({T}_{f}_{T}_{i}\right)}{Δ {T}_{n}}$ umfasst, wobei "k" eine Konstante, "T_{f}" die Endtemperatur, "Tᵢ" die Anfangstemperatur und "ΔTₙ" die erwartete Temperaturänderung ist.

10. System (10) nach einem der Ansprüche 1-9, wobei das Bestimmen des Vollständigkeitsindexes das Bestimmen von $C = k x \frac{\left({Z}_{f}_{Z}_{i}\right)}{Δ {Z}_{n}}$ umfasst, wobei "k" eine Konstante, "Z_{f}" die Endimpedanz, "Zᵢ" die Anfangsimpedanz und "ΔZₙ" die erwartete Impedanzänderung ist.

## Revendications

1. Système médical (10) comprenant :
un générateur (14) comprenant des circuits de traitement (28) configurés pour :
générer au moins une impulsion de champ électrique pulsé, PEF, à délivrer à au moins une électrode (24) d'une pluralité d'électrodes (24), ladite au moins une électrode (24) se trouvant à une extrémité distale (20) d'un cathéter d'ablation PEF (16) et pouvant être positionnée à proximité d'une région cible de tissu à ablater ;
stocker des mesures de paramètres ; et **caractérisé en ce que** ledit générateur est en outre configuré pour déterminer un indice d'exhaustivité indiquant un degré d'exhaustivité de l'ablation de la région cible de tissu, l'indice d'exhaustivité étant déterminé sur la base, au moins en partie, d'une variation d'un paramètre mesuré par rapport à une variation attendue du paramètre mesuré, la variation du paramètre mesuré étant provoquée, au moins en partie, par un degré d'ablation de la région cible.

2. Système (10) selon la revendication 1, dans lequel les circuits de traitement (28) sont configurés pour définir une profondeur, une taille ou une largeur prédéterminée de formation de lésion, et dans lequel l'indice d'exhaustivité comprend un rapport entre une profondeur, une taille ou une largeur atteinte depuis la délivrance de l'énergie PEF au tissu cible par rapport à la profondeur, à la taille ou à la largeur prédéterminée de formation de lésion.

3. Système (10) selon la revendication 1 ou la revendication 2, dans lequel les circuits de traitement (28) sont configurés pour être préprogrammés avec un seuil pour un rapport acceptable d'exhaustivité, et où les circuits de traitement (28) sont configurés pour terminer la génération d'énergie PEF vers le dispositif médical sur la base de l'obtention du seuil pour le rapport acceptable d'exhaustivité.

4. Système (10) selon l'une quelconque des revendications 1 à 3, dans lequel les circuits de traitement (28) sont configurés pour générer une alerte lorsque le rapport déterminé d'exhaustivité dépasse un rapport acceptable prédéterminé de seuil d'exhaustivité.

5. Système (10) selon l'une quelconque des revendications 1 à 4, comprenant en outre un affichage en communication avec les circuits de traitement (28) et le dispositif médical, et dans lequel les circuits de traitement (28) sont configurés pour provoquer l'affichage de l'indice d'exhaustivité pour au moins un sous-ensemble de la pluralité d'électrodes (24).

6. Système (10) selon la revendication 5, dans lequel les circuits de traitement (28) sont configurés pour provoquer l'affichage d'une zone autour de la pluralité d'électrodes (24) traitées avec de l'énergie PEF.

7. Système (10) selon l'une quelconque des revendications 1 à 6, dans lequel les circuits de traitement (28) sont configurés pour provoquer l'affichage d'au moins l'un d'un indicateur codé en couleur et d'une opacité variable sur la base du rapport d'exhaustivité pour chacune de la pluralité d'électrodes (24).

8. Système (10) selon l'une quelconque des revendications 1 à 7, dans lequel les circuits de traitement (28) sont configurés pour mesurer l'impédance d'au moins l'une de chaque électrode (24), entre des électrodes adjacentes (24), entre l'une de la pluralité d'électrodes (24) et une électrode de masse (24).

9. Système (10) selon l'une quelconque des revendications 1 à 8, dans lequel la détermination de l'indice d'exhaustivité comprend de déterminer $C = k x \frac{\left({T}_{f}_{T}_{i}\right)}{Δ {T}_{n}}$ , où « k » est une constante, « T_{f} » est la température finale, « Tᵢ » est la température initiale et « ΔTₙ » est le changement de température attendu.

10. Système (10) selon l'une quelconque des revendications 1 à 9, dans lequel la détermination de l'indice d'exhaustivité comprend de déterminer $C = k x \frac{\left({Z}_{f}_{Z}_{i}\right)}{Δ {Z}_{n}}$ , où « k » est une constante et « Z_{f} » est l'impédance finale, « Zᵢ » est l'impédance initiale, et « ΔZₙ » est le changement attendu d'impédance.
